# EUROPEAN PATENT SPECIFICATION

(11) **EP 3 131 472 B1**
(45) Date of publication and mention of the grant of the patent: **17.03.2021**
(21) Application number: 15718571.1
(22) Date of filing: 16.04.2015
(51) Int. Cl.: A61B 10/00

(54) **FLUID COLLECTION DEVICE**
FLÜSSIGKEITSSAMMELBEHÄLTER
APPAREIL A COLLECTION DE FLUIDE

(30) Priority: 17.04.2014 GB 201407002
(43) Date of publication of application: 22.02.2017
(73) Proprietor: Secretary of State for Health and Social Care, London SW1H 0EU (GB)
(72) Inventor: BISHOP, Oliver, Fittleworth West Sussex RH20 1JE (GB); CHILDS, Ben, Fittleworth West Sussex RH20 1JE (GB)
(74) Representative: Pitchford, James Edward
(86) International application number: PCT/GB2015/051155
(87) International publication number: WO 2015/159089

(56) References cited:
- WO-A1-2005/032377
- GB-A- 2 440 353
- US-A1- 2005 106 753
- US-A1- 2008 193 926
- US-A1- 2009 030 342

## Description

### Field of the Invention

The present invention relates to a fluid collection device, suitable for use in collecting fluids for subsequent analysis or testing. It is particularly applicable, but by no means limited, to the collection of bodily fluids such as oral fluids (e.g. saliva or mucosal excretions), blood or urine.

### Background to the Invention

Typical devices for collecting bodily fluids or such like consist of an absorbent (e.g. sponge) collector head mounted at the end of a shaft. Together, the collector head and the shaft may be referred to as a swab. In use, a user holds the end of the shaft distal from the collector head, e.g. between thumb and forefinger, and manipulates the absorbent collector head to gather a sample of the fluid that is to be analysed or tested (e.g. for DNA analysis, or to test for the presence of certain substances within the fluid, etc.).

Once the sample has been obtained, the swab is typically inserted into a centrifuge tube, the tube is closed, and it is then sent to a test facility (e.g. a lab) for analysis. In the test facility, the centrifuge tube is placed in a centrifuge machine and subjected to a centrifugation process, to cause the fluid to be extracted from the absorbent head of the swab. Once this has been done, a quantity of the extracted fluid is obtained (e.g. using a pipette) and subjected to the test or analysis process for which the sample was initially obtained.

It will be appreciated that the above process is potentially time consuming, in particular due to the centrifugation step. Moreover, in certain circumstances (e.g. "out in the field") a centrifuge machine may not be available. There is therefore a desire to be able to extract the fluid from the absorbent head of a swab in a more efficient manner, without the need for a centrifugation step.

There is also a desire to reduce the likelihood of contamination of the sample during handling, between the time of initially obtaining the sample, and the point of providing a quantity of the sample for the test or analysis process.

Background art is provided in GB 2 440 353 A, WO 2005/032377 A1, US 2009/030342 A1, US 2008/193926 A1 and US 2005/106753 A1.

GB 2 440 353 A discloses an oral fluid collection device comprising an elongate shaft with a swab end. It is further disclosed that the oral fluid collection device may be used in a collection tube which includes a widget that squeezes the swab end on insertion into the tube, causing the oral fluid to be released. In one embodiment the elongate shaft of the swab may be snapped off from the swab end.

WO 2005/032377 A1 discloses a sampling kit which includes a swab with a handle and a sampling head. The kit also includes a container with an optional closure. The sampling head is detachable from the handle by engaging the sampling head with engagement means formed on the closure or on the container, and moving the handle with respect to the engagement means. After detachment of the sampling head from the handle, the sampling head remains in the container. The container may then be sealed using sealing means.

US 2009/030342 A1 discloses a device for sample collection, for releasing a sample material from a sample acquisition device such as a swab. The device for sample collection comprises a housing having an abrasion element, the abrasion element comprising at least one constriction or projection. The sample acquisition device comprises a shank. In certain embodiments a cap for sealing the housing is attached to the shank.

US 2008/193926 A1 discloses a device for extracting a smear sample. The device includes a cavity, into which a sample carrier carrying a smear sample can be introduced. Liquid can be introduced into the cavity through at least one liquid feed connected to the cavity, and the liquid can be removed from the cavity through at least one liquid discharge connected to the cavity. In certain embodiments the sample carrier is in the form of a cotton bud fastened on a carrier stick. On insertion of the sample carrier into the device, the carrier stick is broken off at a weakened fracture point, so that only a short stub of the carrier stick with the cotton bud remains in the device.

US 2005/106753 A1 discloses a device for collecting samples, especially liquid samples to be tested for the presence of an analyte. The collected sample can be stored in the device, and the device can be used to apply, in a drop-wise manner, an aliquot of the sample directly to a test device. The device comprises: a sample container, wherein a portion of said container is flexible; and a sample collector having an absorbent member for collecting a liquid sample, and a dropper assembly which acts as a handle for the absorbent member, the dropper assembly having an orifice for expelling an expressed sample. To express the sample from the absorbent member and through the orifice of the dropper assembly, a user squeezes the flexible portion (e.g. the side walls) of the container.

### Summary of the Invention

The present invention is a fluid collection device as defined in Claim 1 of the appended claims. Also provided is a method of collecting and subsequently dispensing a fluid sample using such a fluid collection device, as defined in Claim 15.

With such a device, a fluid sample gathered by the absorbent collector may be released from the absorbent collector in a simple and efficient manner in the container, without the need for centrifugation.

In certain embodiments the length of the swab and the length of the container are such that the absorbent collector reaches a maximum degree of radial compression at the far end of the container from the opening. Advantageously, this results in the fluid from the absorbent collector being released above the compressed absorbent collector within the container, thereby minimising the likelihood of the fluid being trapped within the container by the compressed absorbent collector.

The internal cross-sectional area of the container decreases smoothly along its length. This reduces the likelihood of the absorbent collector being damaged during the insertion and compression process. The container tapers along its length.

The provision of the closure enables the fluid sample to be sealed within the container, thereby reducing the likelihood of contamination of the sample, or the loss of any of the sample, or the risk of any potentially-hazardous (e.g. infectious) constituents within the sample being passed on to others.

In certain embodiments, the closure comprises friction-fit means (for example a plurality of radially-disposed gripping fins) by which the shaft is attached. Such friction-fit means facilitate assembly of the fluid collection device. However, other means for attaching the closure to the shaft are also possible, such as using adhesive or a threaded fastening arrangement, as those skilled in the art will appreciate.

In certain embodiments the opening of the container is provided with a screw thread, and the closure is provided with a complementary screw thread. However, other means for engaging the closure with the container are also possible, as those skilled in the art will appreciate.

In certain embodiments the internal cross-sectional area of the container decreases along its length in such a manner that, in use, the absorbent collector is further radially compressed as the closure is engaged with the container. Thus, the act of effecting the engagement of the closure with the container (e.g. by screwing the closure to the container) causes further fluid to be squeezed out of the absorbent collector. With a screw-threaded closure arrangement, this also affords a mechanical advantage, making it easier to further compress the absorbent collector.

The closure further comprises an outlet through which, in use, fluid within the container can be dispensed, subsequent to the closure having been engaged with the container. Such an outlet advantageously enables fluid released from the absorbent collector to be dispensed without needing to undo the closure to the container, thereby reducing the likelihood of contamination of the sample during handling. Also, by not needing to undo the closure in order to dispense fluid from the container, this prevents reversal of the compressive effect to which the absorbent collector was subjected during the process of engaging the closure with the container. That is to say, this mitigates against the absorbent collector reabsorbing at least some of the released fluid, which would otherwise be likely to happen were the closure to be undone and, in so doing, the absorbent collector moved into a position (towards the opening of the container) at which its degree of radial compression would be less and its absorbency would consequently be greater.

For example, the closure may further comprise a spout in which the outlet is formed. In certain embodiments, such a spout enables the device to function as a dropper, for dispensing the fluid released from the absorbent collector in a drop-by-drop manner. In the case of such a dropper, the size (e.g. diameter) of the outlet determines the quantity of fluid that is dispensed in each drop (for a given viscosity of liquid).

In certain embodiments, the closure further comprises means for reversibly closing and opening the outlet, thereby reducing the likelihood of contamination of the sample during handling and ensuring that the container does not leak (e.g. during transit).

In a presently-preferred embodiment, the means for reversibly closing and opening the outlet comprises a removable cap that is attached to the rest of the closure by a flexible tether. Such a tether prevents the cap from being lost when it is detached from the outlet. The cap may have a protruding lip, thereby facilitating removal of the cap from the outlet. Further the cap may comprise plug means configured to engage within the outlet, to enhance the sealing of the outlet. The cap and/or closure may also comprise detent means for securing the cap in place when it closes the outlet.

The closure, including the cap and the flexible tether, may be formed as a unitary construction, for example by injection moulding a plastics material.

In certain embodiments the container is formed of a squeezable material (for example a plastics material, for example by injection moulding) to enable the user to apply squeezing pressure to the collection tube when dispensing the fluid released from the absorbent collector. Such squeezable material is particularly beneficial if the closure is provided with a spout so as to enable the device to function as a dropper. Further, the thickness of the wall of the container may be greater around the far end of the container from the opening (thereby enhancing the ability of the container in that region to compress the absorbent collector), and thinner elsewhere (thereby providing a region that is easier for the user to squeeze).

In certain embodiments the fluid collection device is for the collection of bodily fluids such as oral fluids, blood or urine. However, applications in many other areas of industry or research are also possible.

### Brief Description of the Drawings

Embodiments of the invention will now be described, by way of example only, and with reference to the drawings in which:
Figure 1 illustrates (i) a swab part having an absorbent collector head at one end and a closure assembly at the other end, the closure assembly comprising a threaded (screw-on) closure part and a dropper spout with a removable (push-off) cap; (ii) a tapered collection tube having a threaded opening; and (iii) an optional container of buffer fluid;
Figures 2a to 2f illustrate the apparatus of Figure 1 as in use for the collection and subsequent dispensing of a sample of bodily fluid, wherein

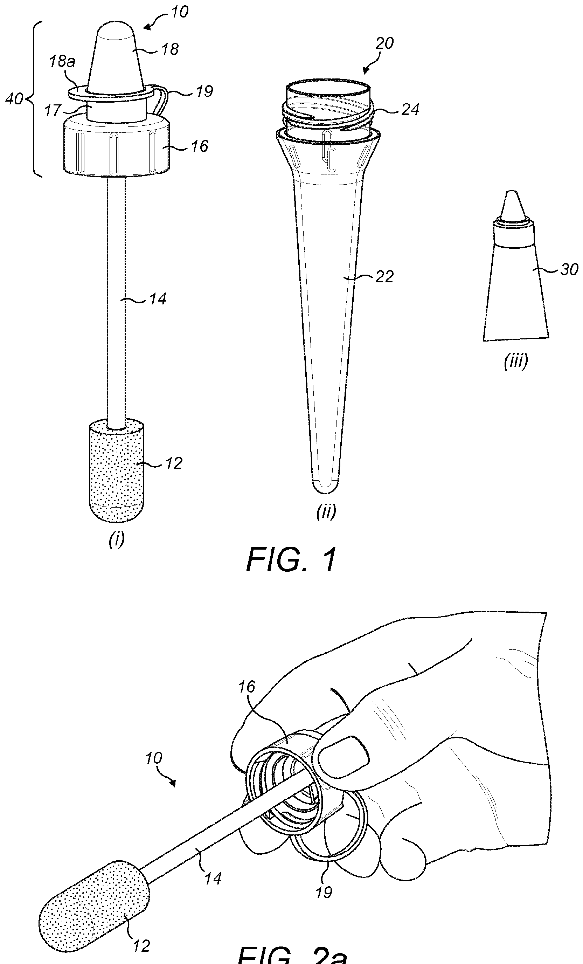

Figure 2a illustrates the swab part as in use for the collection of a sample of bodily fluid,

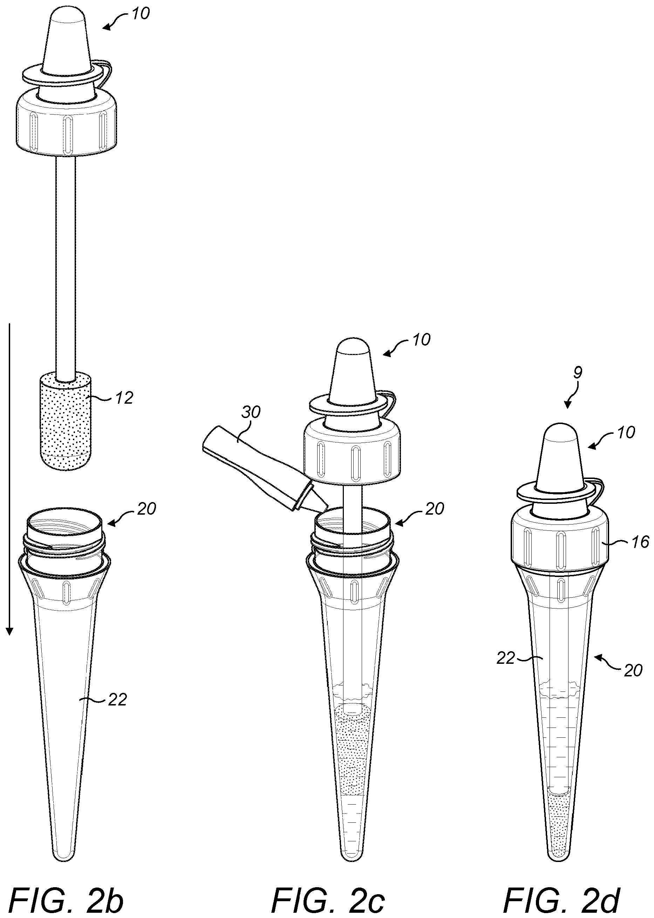

Figure 2b illustrates the swab part being inserted into the collection tube,
Figure 2c illustrates the addition of buffer fluid into the collection tube containing the swab part,
Figure 2d illustrates the threaded (screw-on) closure of the swab part having being screwed onto the threaded opening of the collection tube,

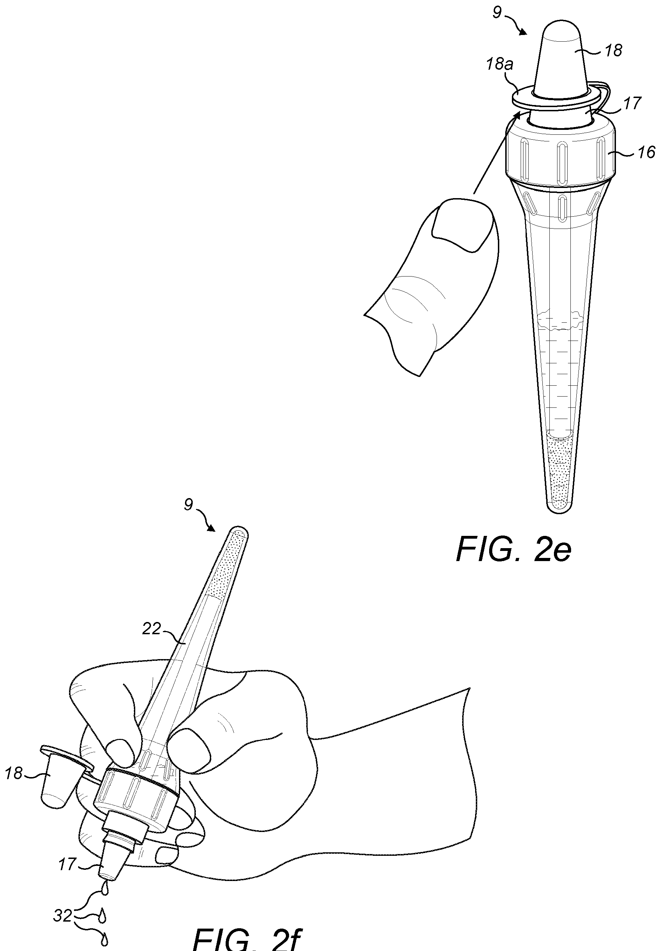

Figure 2e illustrates a user in the process of pushing-off the removable cap from the closure, to open the dropper spout, and
Figure 2f illustrates the apparatus of Figure 2e having been inverted by the user, with the cap having been removed from the closure, and the user applying gentle squeezing pressure to the collection tube to cause some drops of its contents to be dispensed;

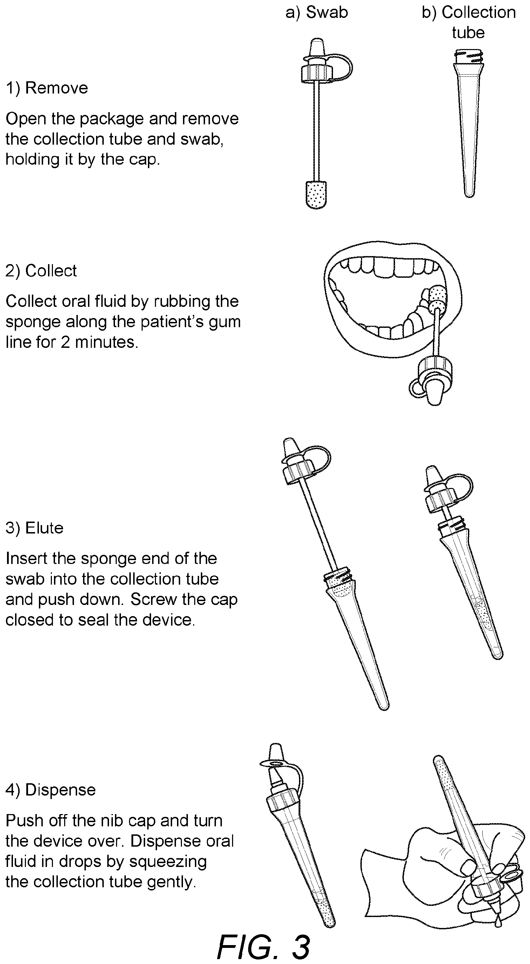

Figure 3 shows an example set of user instructions for the apparatus of Figure 1;

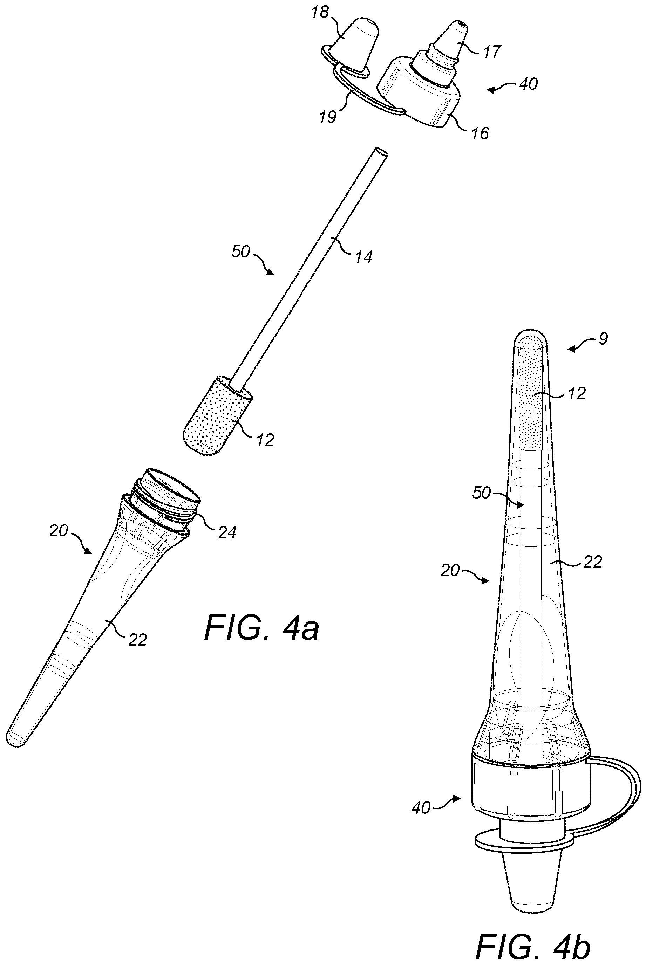

Figure 4a is an exploded schematic diagram of the components of the swab part and the collection tube of Figure 1;
Figure 4b illustrates the components of Figure 4a rejoined, with the closure of the swab part having being screwed onto the threaded opening of the collection tube;

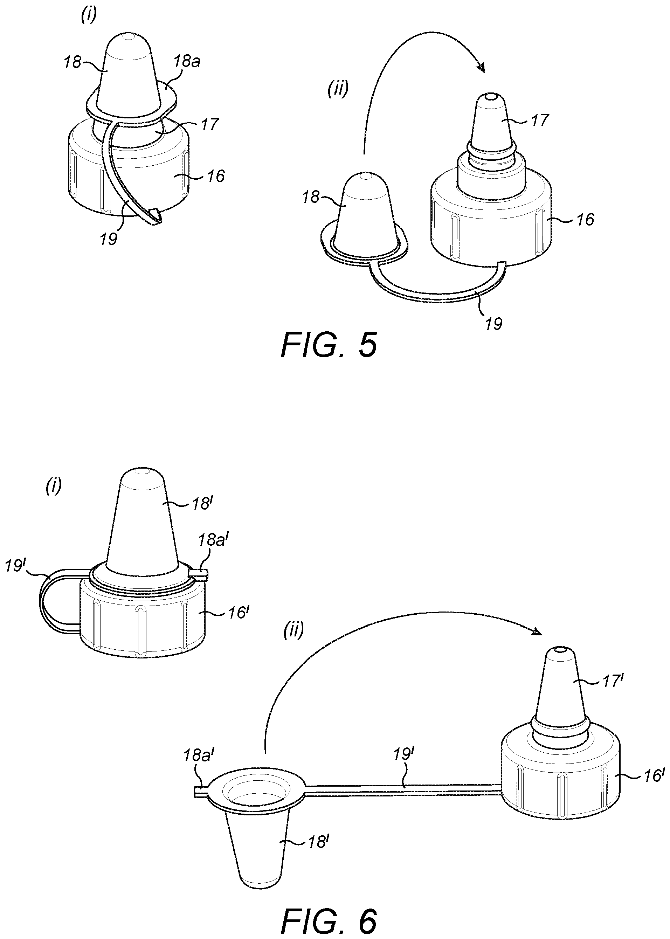

Figure 5 illustrates (i) the closure of Figure 1 with the dropper spout in a closed configuration; and (ii) the closure of Figure 1 with the dropper spout in an open configuration (as manufactured);
Figure 6 illustrates (i) an alternative closure with the dropper spout in a closed configuration; and (ii) said alternative closure with the dropper spout in an open configuration (as manufactured);

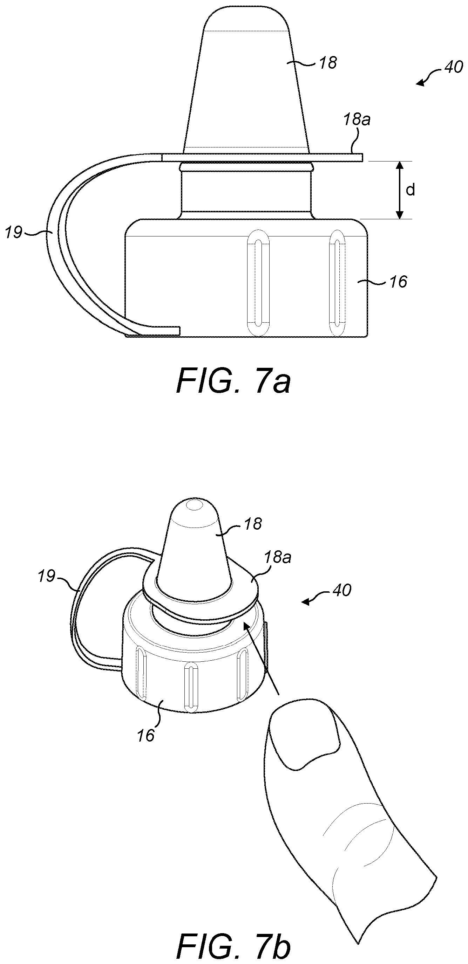

Figure 7a illustrates a side view of the closure of Figure 1 with the dropper spout in a closed configuration;
Figure 7b illustrates the closure of Figure 7a, with the dropper spout in the process of being opened;

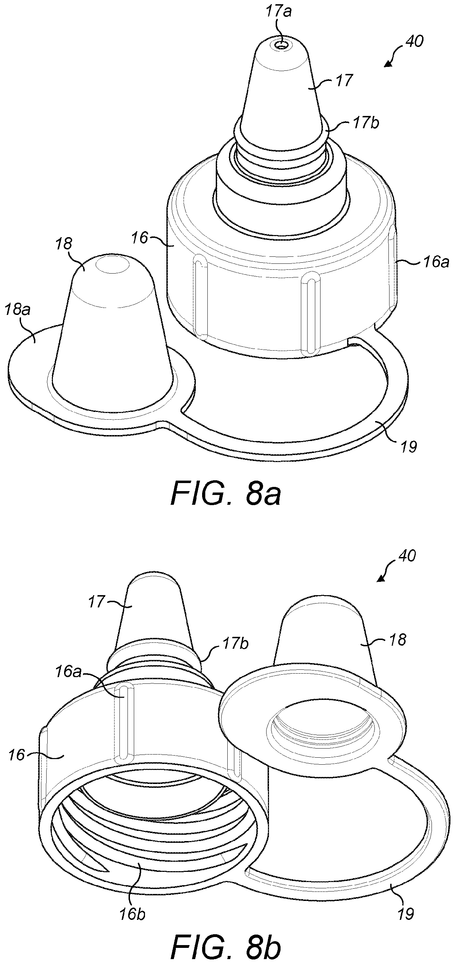

Figures 8a and 8b are perspective illustrations of the closure of Figure 1;

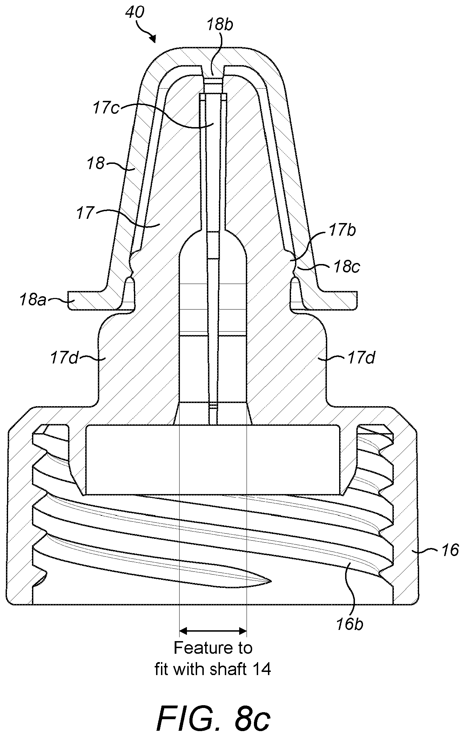

Figure 8c is a cross-sectional view of the closure of Figure 1, with the dropper spout in the closed configuration;

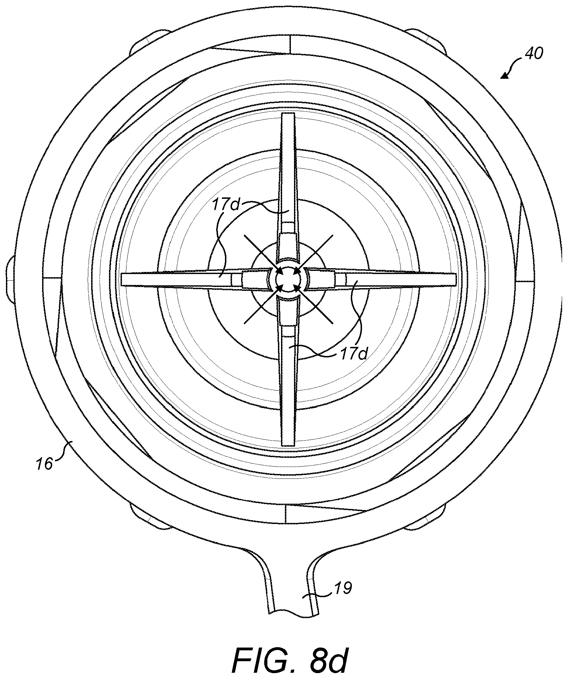

Figure 8d illustrates the underside of the closure of Figure 1, looking up into the closure from underneath and thereby showing its internal structure;

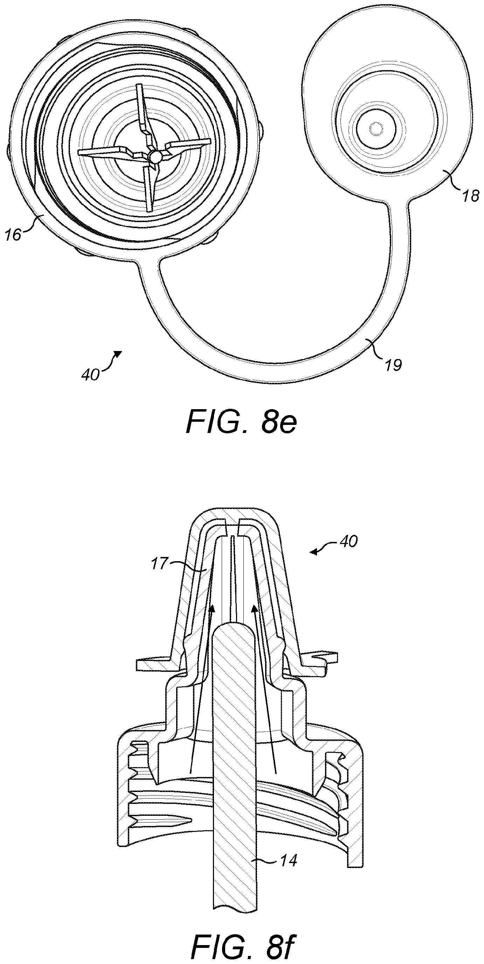

Figure 8e is a further illustration of the underside of the closure of Figure 1;
Figure 8f is a cross-sectional view of the closure of Figure 1 with the swab shaft attached, and illustrating how fluid can pass the end of the swab shaft in use;

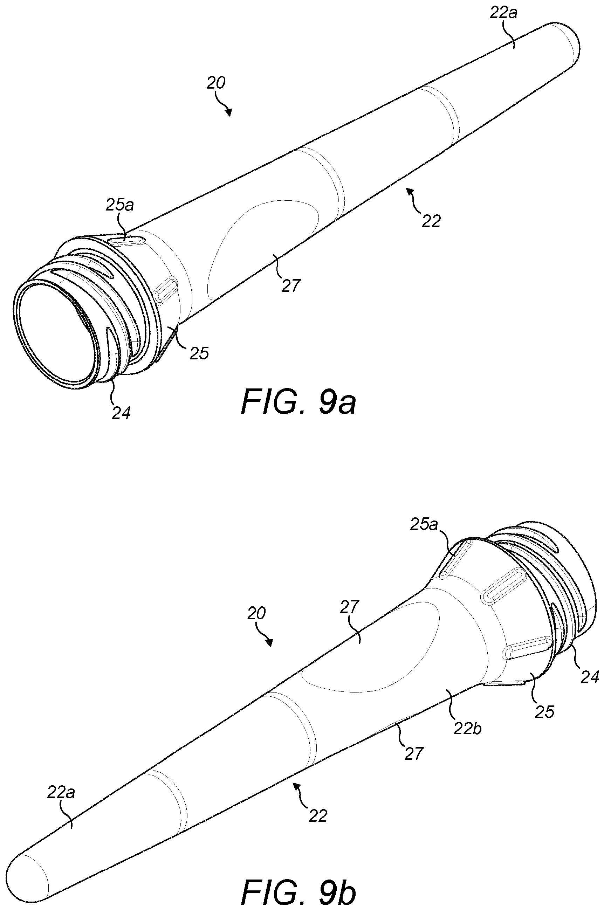

Figures 9a and 9b are perspective illustrations of the collection tube of Figure 1;

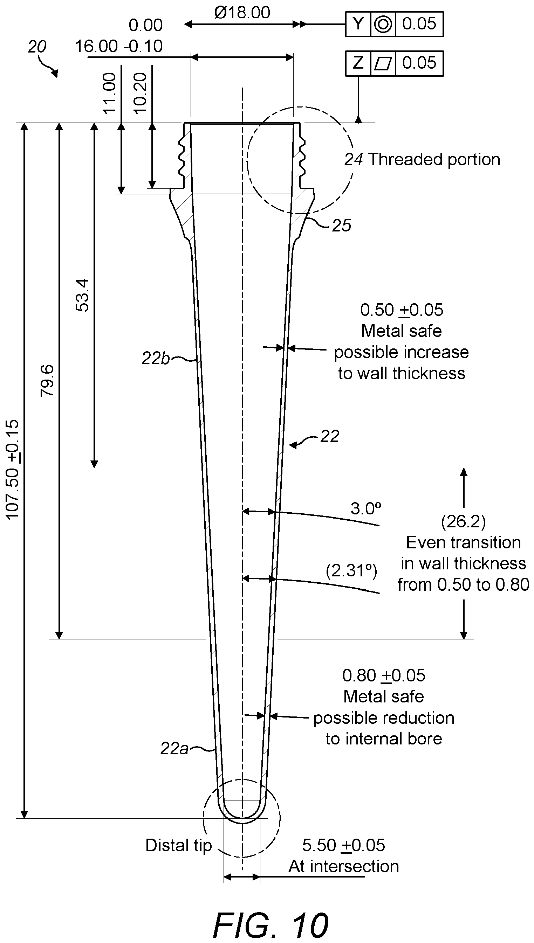

Figure 10 is a longitudinal cross-sectional view of the collection tube of Figure 1, with possible dimensions (in millimetres) by way of example only, and showing a non-uniform thickness of the wall of the collection tube;

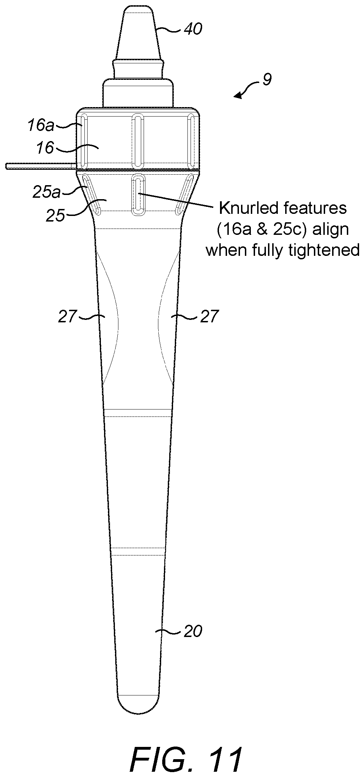

Figure 11 is a side view of the closure and collection tube of Figure 1, with the closure having being screwed onto the collection tube;

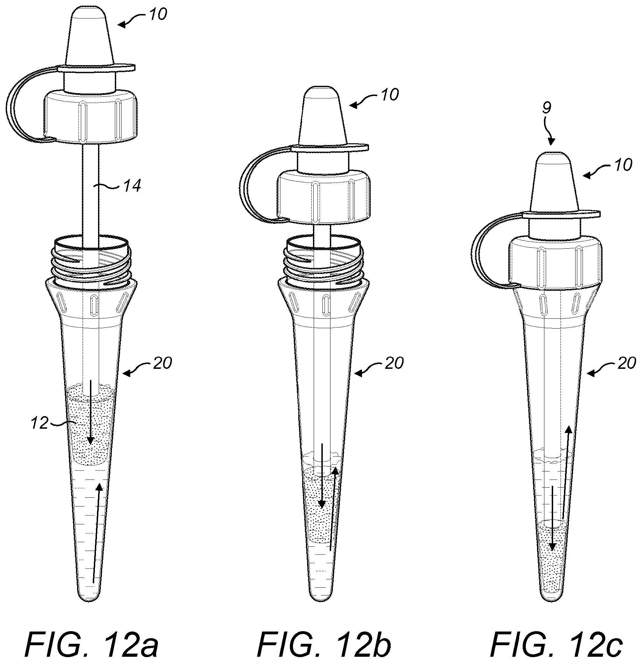

Figure 12 illustrates the process by which a maximum available quantity of fluid is released from the absorbent collector head of Figure 1, upon the collector head reaching a maximum degree of compression at the bottom of the tapered collection tube, and an associated flushing effect obtained through the absorbent head; and

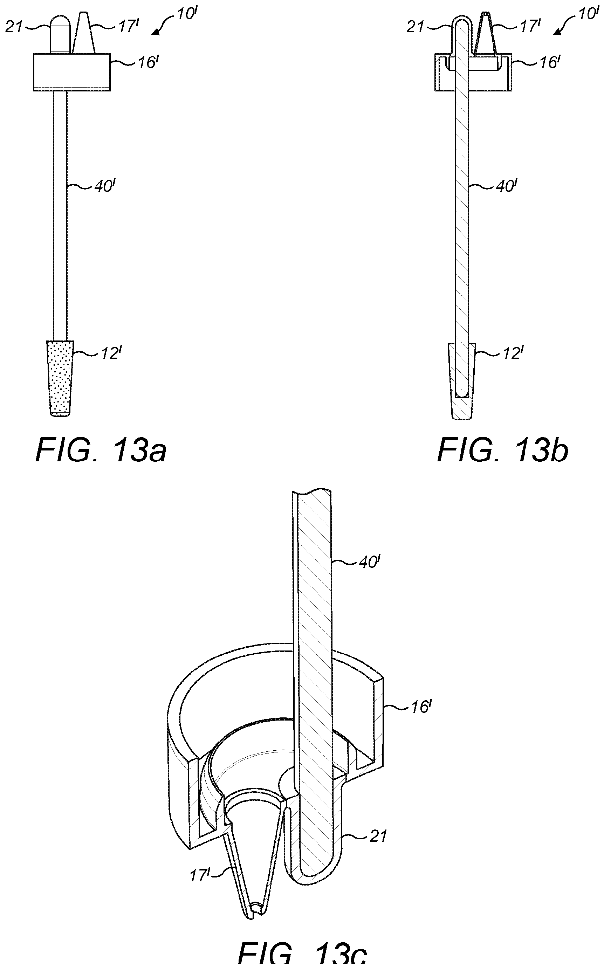

Figures 13a-13c illustrate a possible alternative arrangement for the attachment of the swab shaft to the closure, with Figure 13a showing a side view, Figure 13b showing a side view in cross section, and Figure 13c showing a close-up perspective cross-sectional view of the attachment arrangement.

In the figures, like elements are indicated by like reference numerals throughout.

### Detailed Description of Preferred Embodiments

The present embodiments represent the best ways known to the applicants of putting the invention into practice. However, they are not the only ways in which this can be achieved.

In the description which follows (and in the discussion of the drawings above), embodiments of fluid collection devices are described primarily in relation to the collection of bodily fluids, and oral fluids in particular. However, the invention is not limited to such applications, and it should be appreciated that embodiments may be produced for the collection of fluids in many other areas of industry or research (such as, but in no way limited to, the fields of biotechnology, biochemistry, chemistry, food science, beverages, lubricants, petrochemicals, agrochemicals, marine science, forensic science and investigation, etc.).

### Overview

By way of an initial overview, as illustrated in Figure 1(i)-(ii), a fluid collection device according to an embodiment of the invention comprises a swab part 10 and an elongate collection tube or container 20.

Figure 1(i) illustrates the swab part 10 having a substantially rigid shaft 14 with an absorbent collector head 12 at one end. The collector head 12 is made of foam or sponge, for example, or some other absorbent material that is able to absorb a fluid and to release the fluid when the material is subsequently compressed. As illustrated, the collector head 12 is substantially cylindrical in shape, and may also have a rounded tip (for comfort if used orally). The shaft 14 can be made of any suitable material. Preferably the shaft 14 is made of a plastics material.

Attached to the other end of the shaft 14 is a closure assembly 40 comprising a threaded (screw-on) closure part 16 and a dropper spout 17 with a reversibly-removable (push on / push or pull off) cap 18. The cap 18 incorporates a protruding lip 18a with which a user can urge the cap 18 off the dropper spout 17. The cap 18 is attached to the closure part 16 via a flexible tether 19.

The closure assembly 40, including the closure part 16, the dropper spout 17, the cap 18 and the flexible tether 19, is preferably formed as a unitary construction from a plastics material, for example by injection moulding.

The dropper spout 17 comprises a fluid-conveying channel which extends from the underside of the closure part 16 to an opening (17a, Figure 8a) at the tip of the dropper spout 17. The opening, which serves as an outlet for fluid held within the collection tube 20 in use, is uncovered when the cap 18 is removed, thereby putting the opening into fluid communication with the underside of the closure part 16. As described in greater detail below, the shaft 14 of the swab is attached to the underside of the closure assembly 40 in such a manner as to permit such fluid communication.

As illustrated in Figure 1(ii), the collection tube 20 comprises a tapered body portion 22 and a threaded closure portion 24 proximal to the opening of the tube. The threaded closure portion 24 of the collection tube 20 is formed so as to screwably engage in a fluid-tight manner with the threaded closure part 16 of the closure assembly 40 of the swab part 10, with the shaft 14 and absorbent head 12 of the swab part 10 going inside the body portion 22 of the collection tube 20.

The body 22 of the collection tube 20 is smoothly tapered such that, when the shaft 14 and absorbent head 12 of the swab part 10 are inserted into the collection tube 20, the absorbent head 12 is gradually compressed (undergoing radial compression) as it progresses down the inside of the body 22 of the collection tube 20. That is to say, the extent of radial compression of the absorbent head 12 increases as the absorbent head 12 progresses down the inside of the tapered body 22. This process of compression of the absorbent head 12 continues until the closure part 16 of the closure assembly 40 of the swab part 10 has fully engaged with the closure portion 24 of the collection tube 20 (i.e. the closure part 16 and the closure portion 24 are fully screwed together).

Preferably, at the point of full engagement of closure part 16 with closure portion 24, the degree of radial compression of the absorbent head 12 reaches a maximum level (the term "maximum" being relative to the degree of compression experienced by the absorbent head 12 during the preceding stages of the insertion/closure process, and not necessarily the absolute maximum degree of compression of which the absorbent head 12 is in principle capable). Accordingly, when the closure part 16 and closure portion 24 are fully engaged, the ability of the absorbent head 12 to retain liquid reaches a minimum level (again, the term "minimum" being relative to the liquid retention ability of the absorbent head 12 during the preceding stages of the insertion/closure process).

Thus, in use, as a consequence of this compression of the absorbent head 12 during the insertion/closure process, a fluid sample that has been collected using the swab part 10 (i.e. has been absorbed by the absorbent head 12) is released from (i.e. squeezed out of) the absorbent head 12, whereupon it exists as free liquid within the body 22 of the collection tube 20.

The internal length and tapering shape of the collection tube 20, and the overall length of the swab part 10, are such that the absorbent head 12 reaches its state of maximum compression at the bottom of the inside of the collection tube 20, thereby causing the fluid that is released from the absorbent head 12 to go above the compressed absorbent head 12, within the body 22 of the collection tube 20. In practice, as the absorbent head 12 is pushed down into the collection tube 20, displaced air pushes up through the absorbent head 12, and the fluid is released above the compressed absorbent head 12. This process, by which a maximum available quantity of fluid is released from the compressed absorbent head 12 when the absorbent head 12 reaches its state of maximum compression (at the bottom of the inside of the collection tube 20, at the point of full engagement of closure part 16 with closure portion 24), accompanied by a flushing effect due to the displaced air (or, in some cases, buffer fluid) being forced up through the absorbent head 12, is discussed in greater detail below with reference to Figure 12.

As those skilled in the art will appreciate, in order to achieve the above-described radial compression of the absorbent head 12, an important property of the collection tube 20 is that its internal cross-sectional area becomes smaller along the length of the tube body 22 (i.e. in a longitudinal direction, away from the opening through which the absorbent head 12 is inserted). Preferably the internal cross-sectional area of the tube 20 reaches a minimum at or near the bottom of the tube 20. Preferably the internal cross-sectional area of the tube 20 gradually decreases along the length of the tube, i.e. tapers smoothly, thereby causing the absorbent head 12 to be gradually squeezed during the insertion and compression process, and thereby avoiding damage to the absorbent head 12 during the insertion and compression process.

In alternative examples outside the scope of the present invention, the internal cross-sectional area may change in a less smooth, more pronounced, manner (e.g. stepwise).

As illustrated, preferably the external cross-sectional dimension (e.g. outside diameter) of the body 22 of the collection tube 20 changes (e.g. tapers) in a manner that substantially corresponds to the change in internal cross-sectional area of the collection tube 20.

The collection tube 20 is preferably formed from a plastics material, for example by injection moulding. In a particularly preferred embodiment the collection tube 20 is formed of a transparent or translucent plastics material, to enable a user to see (at least opaquely) the contents of the collection tube 20.

When the cap 18 of the closure assembly 40 is attached over the dropper spout 17 (i.e. the dropper spout 17 is in a closed configuration) and the closure part 16 of the closure assembly 40 is screwed down onto the closure portion 24 of the collection tube 20, the interior of the collection tube 20 is sealed in a fluid-tight manner.

With the closure part 16 of the closure assembly 40 remaining attached to the closure portion 24 of the collection tube 20, when the cap 18 of the closure assembly 40 is removed from the dropper spout 17 (i.e. the dropper spout 17 is in an open configuration), the interior of the collection tube 20 is put into fluid communication with the tip of the dropper spout 17, enabling fluid within the collection tube 20 to be dispensed through the dropper spout 17. Preferably such dispensing is effected by a user applying gentle squeezing pressure to the collection tube 20.

Optionally, as shown in Figure 1(iii), a container of buffer fluid 30 may be provided along with the swab part 10 and collection tube 20.

The swab part 10 and collection tube 20 are preferably supplied to the user wrapped in sterile packaging. For instance, a swab part 10 and a collection tube 20 may be supplied as a pair, with the swab part 10 and collection tube 20 being separated and packaged side-by-side in a flow wrap. The buffer fluid 30 may be supplied separately.

### Method of use

Figures 2a to 2f illustrate the apparatus of Figure 1 as in use for the collection and subsequent dispensing of a sample of bodily fluid, for example. Reference is also made to Figure 3, which provides an example set of user instructions for the apparatus.

To begin with, Figure 2a illustrates the swab part 10 in use for the collection of such a sample. The user (e.g. a clinician) removes the swab part 10 from its packaging, holding the swab part 10 by the outside of the closure part 16 (so as to avoid contaminating the shaft 14 or the absorbent collector head 12), and manipulates the swab so that the absorbent head 12 absorbs a sample of the fluid that is desired to be analysed or tested. For example, as shown in Figure 3, a sample of oral fluid may be collected by rubbing the absorbent head 12 along a gum line for a couple of minutes.

The swab part 10 is then inserted into the collection tube 20, as illustrated in Figure 2b (the arrow indicating the direction of motion of the swab part 10 relative to the collection tube 20). As the absorbent head 12 of the swab part 10 progresses down the inside of the tapered body 22 of the collection tube 20 the absorbent head 12 is gradually compressed, and the fluid that had been collected in the absorbent head 12 is squeezed out (or "eluted"), into the body 22 of the collection tube 20.

Depending on the nature of the sample and the circumstances of the test, buffer fluid 30 can be added into the collection tube 20 containing the swab part 10, as illustrated in Figure 2c. In such a case, the buffer fluid 30 is preferably added on top of the absorbent head 12 before the closure part 16 is screwed down, thereby optimising the mixing of the buffer fluid with the fluid collected by the absorbent head 12. Further, by adding the buffer fluid before the absorbent head 12 is fully inserted into the collection tube 20, i.e. such that the absorbent head 12 passes down through at least some of the buffer fluid as the absorbent head 12 is pushed towards the bottom of the collection tube 20, this advantageously gives rise to a flushing effect in respect of the collected fluid, as displaced buffer fluid is forced up through the absorbent head 12. This flushing effect, to which the collected fluid is subjected, is described in greater detail below with reference to Figure 12.

As illustrated in Figure 2d, the threaded closure part 16 of the swab part 10 is then screwed fully down onto the threaded closure portion 24 of the collection tube 20, resulting in a fully closed assembly 9. By reaching full engagement of closure part 16 with closure portion 24, this causes maximum compression of the absorbent head 12 at the bottom of the body 22 of the collection tube 20, and the maximum available release of the collected fluid into the body 22 of the collection tube 20, above the compressed absorbent head 12. That is to say, the act of screwing the closure part 16 fully onto the collection tube 20 causes further fluid to be squeezed out of the absorbent head 12. The screwing mechanism also gives the user a mechanical advantage, potentially allowing them to compress the absorbent head 12 by more than would otherwise be possible if the screwing mechanism were not provided, and thereby enabling more fluid to be released from the absorbent head 12.

As shown in Figure 2e, when the user wishes to dispense fluid from within the closed assembly 9, he applies upward pressure against the protruding lip 18a of the cap 18, to urge the cap 18 off the dropper spout 17 and thereby open up the tip of the dropper spout 17 (which is in communication with the interior of the collection tube 20). This application of pressure to the protruding lip 18a of the cap 18 may be done, for example, by pushing a finger or thumb at least partly into the gap between the protruding lip 18a and the top of the closure part 16 (this gap typically being smaller than the thickness of a typical finger or thumb) and thereby urging the cap 18 away from the dropper spout 17

With the cap 18 having been removed from the dropper spout 17 (but remaining attached to the closure part 16 by means of the flexible tether 19), the user then inverts the closed assembly 9, as shown in Figure 2f, holding the body 22 of the collection tube in a similar manner to a marker pen.

The user then applies gentle squeezing pressure to the body 22 of the collection tube, causing drops 32 of the collected fluid to be dispensed through the dropper spout 17, drop by drop.

Using the present apparatus, by the user holding the swab part 10 by the outside of the closure part 16 (as per Figure 2a), gathering a fluid sample using the absorbent head 12 of the swab part 10, inserting the swab part 10 into the collection tube 20 (as per Figure 2b), and engaging the closure part 16 of the swab part 10 with the closure portion 24 of the collection tube 20 (Figure 2d), the fluid sample is transferred into the collection tube 20 without any human contact with the sample, thereby minimising the possibility of contaminating the sample during handling. Further, by dispensing the fluid sample from the closed assembly 9 via the dropper spout 17 (as per Figure 2f) the fluid sample can be provided for test or analysis purposes, again without any human contact with the sample. In this manner, with the presently-preferred embodiment, typically between about 0.5 ml and about 1 ml of collected fluid may be extracted from the swab and subsequently dispensed for test or analysis purposes.

### Manufacturing and design details

Figure 4a is an exploded schematic diagram of the components used to form the swab part 10 of the presently-preferred embodiment of the fluid collection device. The collection tube 20 is also shown alongside.

In the presently-preferred embodiment the closure assembly 40, including the closure part 16, the dropper spout 17, the cap 18 and the flexible tether 19, is formed as a unitary construction from a plastics material by injection moulding.

The shaft 14 and the absorbent collector head 12 are obtained prefabricated and pre-connected, as article 50. To form the overall swab part 10, the end of the shaft 14 distal from the collector head 12 is attached to the underside of the closure part 16, in a manner that is described in more detail below.

Figure 4b illustrates the components of Figure 4a rejoined, with the closure part 16 of the swab part 10 having being screwed onto the threaded closure portion 24 of the collection tube 20. From this it can be seen that, when the closure part 16 and the closure portion 24 are fully screwed together, the absorbent head 12 is fully compressed at the far end of the tapered body 22 of the collection tube 20 (the far end being the narrowest part of the collection tube 20).

Turning to the closure assembly 40 in more detail, Figure 5 illustrates (i) the closure assembly 40 with the dropper spout 17 in a closed configuration, with the cap 18 over the dropper spout 17; and (ii) the same closure assembly 40 with the dropper spout 17 in an open configuration.

As mentioned above, in the presently-preferred embodiment the closure assembly is manufactured from a plastics material by injection moulding. In the open configuration of Figure 5(ii) it can be seen that the closure assembly 40 is formed such that the cap 18 is oriented in the same direction as the dropper spout 17, with the cap 18 being attached to the closure part 16 by the thin flexible tether 19. The protruding lip 18a of the cap 18 is in the same plane as the flexible tether 19. When attaching the cap 18 over the dropper spout 17 (as indicated by the curved arrow in Figure 5(ii)), so as to close the spout 17, the tether 19 flexes up and around to enable the cap 18 to close the spout 17. By virtue of the cap 18 and the spout 17 being oriented in the same direction, this facilitates manufacture of the closure assembly 40 by injection moulding, as the entirety of the closure assembly 40 can be removed from a mould in a single direction.

To further illustrate this advantageous design aspect of the closure assembly 40, Figure 6 illustrates (i) an alternative closure assembly 40' with the dropper spout 17' in a closed configuration; and (ii) the same alternative closure assembly 40' with the dropper spout 17' in an open configuration. In this variant, the cap 18' is oriented in the opposite direction to the dropper spout 17'.

As with the embodiment of Figure 5, the cap 18' is attached to the closure part 16' by a thin flexible tether 19', and a protruding lip or tab 18a' (to assist the user when lifting the cap 18' off the dropper spout 17', when opening the spout 17) is in the same plane as the flexible tether 19'. When attaching the cap 18' over the dropper spout 17' (as indicated by the curved arrow), so as to close the spout 17', the tether 19' bends in the manner shown in Figure 6(ii), to enable the cap 18' to close the spout 17'.

However, with this embodiment of Figure 6, since the cap 18' and the spout 17' are oriented in opposite directions, manufacture of the closure assembly 40' by injection moulding is not as straightforward as in the case of the embodiment of Figure 5, as the entirety of the closure assembly 40' cannot be removed from a mould in a single direction. The embodiment of Figure 5 is therefore preferable, to facilitate manufacture.

Figure 7a illustrates a side view of the closure assembly 40 (e.g. of Figures 1 and 5) with the dropper spout 17 in a closed configuration, and Figure 7b illustrates the same closure assembly 40 with the dropper spout 17 in the process of being opened. Figures 7a and 7b illustrate that the gap 'd' between the protruding lip 18a of the cap 18 and the top of the closure part 16 is designed to be sufficient to enable a finger or thumb to be at least partly inserted into that gap, to enable the cap 18 to be urged off the dropper spout 17.

Figures 8a and 8b are perspective illustrations of the closure assembly 40, showing certain constructional details more closely. A number of knurled features or longitudinal ribs 16a are provided around the outside of the closure part 16. These ribs 16a enhance the user's grip of the closure part 16 when screwing it onto the closure portion 24 of the collection tube 20.

Further, when the closure part 16 has fully engaged with the closure portion 24 (i.e. the closure part 16 and the closure portion 24 are fully screwed together) the ribs 16a of the closure part 16 align with corresponding ribs 25a provided around the shoulder 25 of the collection tube 20 (as discussed below and illustrated in Figures 9a, 9b and 11), thereby providing a visual indication to the user as to the point at which the closure part 16 is fully engaged with the closure portion 24, and consequently the absorbent head 12 is fully compressed at the bottom of the tapered body 22 of the collection tube 20.

Figure 8a also illustrates the opening 17a at the tip of the dropper spout 17, this opening 17a being at the end of a fluid-conveying channel which extends through to the underside of the closure part 16. (This fluid-conveying channel is denoted by 17c in Figure 8c, which illustrates a cross-sectional view of the closure assembly 40, including the dropper spout 17.)

Threads 16b are provided around the inside of the closure part 16, as illustrated in Figure 8b, for engagement with the threaded closure portion 24 of the collection tube 20.

Further, Figures 8a and 8b also illustrate a protruding ridge 17b formed around the outside of the dropper spout 17, this ridge 17b being shaped and configured to reversibly engage with a corresponding feature formed around the inside of the cap 18, thereby acting as a detent and helping to secure the cap 18 in place when on the spout 17.

Turning now to Figure 8c, this shows a cross-sectional view of the closure assembly 40 with the cap 18 on top of the dropper spout 17, the dropper spout thereby being in the closed configuration. As illustrated, in the presently-preferred embodiment, a protruding plug 18b is provided on the inside of the cap 18, to reversibly engage with the opening 17a at the tip of the dropper spout 17 in a fluid-tight manner when the cap 18 is on the spout 17.

Further, a ridge 18c is provided around the inside of the cap 18, to reversibly engage with the corresponding ridge 17b formed around the outside of the dropper spout 17. With the embodiment illustrated, the cap 18 is designed to flex slightly to enable ridge 18c to pass over ridge 17b as the cap 18 is fitted onto the spout 17, such that ridge 18c ultimately locates just beyond ridge 17b and acts as a detent. In an alternative embodiment, ridge 18c may be replaced with a recess, such a recess engaging with ridge 17b. In another alternative embodiment, ridge 17b may be replaced with a recess, such a recess engaging with ridge 18c.

Figure 8c also illustrates the fluid-conveying channel 17c extending from the opening 17a at the tip of the dropper spout 17 through to the underside of the closure part 16. The channel 17c is opened when the cap 18 is removed from the tip of the dropper spout 17, thereby putting the opening 17a into fluid communication with the underside of the closure part 16.

Further, Figure 8c also illustrates opposing members 17d within the closure assembly, that are shaped and configured to receive and retain the shaft 14 during assembly of the swab part 10, whilst not blocking the fluid-conveying channel 17c.

Figure 8d provides a close-up view of the underside of the closure part 16 of the closure assembly 40, looking up into the closure part 16 from underneath and thereby showing the internal structure of the closure assembly 40. From this, it can be seen that, in the presently-preferred embodiment, four shaft-retaining members or fins 17d are provided, arranged in a cruciform manner at 90 degree intervals around the position of the shaft 14, to support/suspend the shaft 14 whilst leaving gaps for fluid from the collection tube 20 to enter and flow down the fluid-conveying channel 17c in use. During the manufacture of the swab part 10, the distal end of the shaft 14 (of article 50 of Figure 4a) is inserted between the fins 17d in a friction-fit manner, with the fins 17d gripping the shaft 14 with sufficient force to prevent the shaft 14 from being detached (i.e. pulled out) during use (e.g. when collecting oral fluid inside a patient's mouth). The fins 17d grip the shaft 14 by being radially the same distance apart as, or slightly closer together than, the diameter of the shaft 14. This results in a compressive force being exerted on the shaft 14, increasing the friction at the points of contact. As those skilled in the art will appreciate, the closer together the fins 17d, the greater the compressive force that is exerted on the shaft 14.

The fins 17d suspend the shaft 14 within the closure part 16 and, in use, allow fluid to flow through the spaces between the respective fins 17d, and thence around and past the end of the shaft 14 and into the fluid-conveying channel 17c of the dropper spout 17. The four small inwardly-pointing diagonal arrows in the centre of Figure 8d illustrate that, when the inside of the closure part tapers towards the dropper tip, the fluid can flow through the small gaps between the ends of the fins 17d.

Figure 8e is a further illustration of the underside of the closure assembly 40; showing the cruciform arrangement of the fins 17d within the closure 16.

Figure 8f is a cross-sectional view of the closure assembly 40 (in a closed configuration), with the upwardly-pointing arrows illustrating how fluid can pass the end of the swab shaft 14 to reach the tip of the dropper spout 17.

Figures 9a and 9b show perspective illustrations of the collection tube 20, and Figure 10 provides a longitudinal cross-sectional view of the collection tube of the collection tube 20, with possible dimensions (in millimetres) by way of example only.

In the presently-preferred embodiment the collection tube 20 is formed from a transparent or translucent plastics material by injection moulding. The use of a transparent or translucent plastics material enables a user to see (at least opaquely) the contents of the collection tube 20. Further, a calibrated volume scale may be marked on the collection tube 20 to enable the user to quantify the volume of the fluid held therein.

A shoulder 25 is formed beneath the threaded closure portion 24, the shoulder 25 providing a surface for the closure part 16 of the swab part 10 to but against when the closure part 16 and the closure portion 24 are screwed together. Further, a number of knurled features or longitudinal ribs 25a are provided around the shoulder 25, corresponding in number and position to the ribs 16a provided around the outside of the closure part 16. The ribs 25a enhance the user's grip of the collection tube 20 when screwing together the collection tube 20 and the closure part 16 of the swab part 10.

Further, as mentioned above, the ribs 25a provided around the shoulder 25 of the collection tube 20 are positioned so as to align with the ribs 16a provided around the outside of the closure part 16 when the closure part 16 and the closure portion 24 are fully screwed together (as shown in Figure 11), thereby providing a visual indication to the user as to the point at which the closure part 16 is fully engaged with the closure portion 24, and consequently the absorbent head 12 is fully compressed at the bottom of the collection tube 20.

The body 22 of the collection tube 20 is smoothly tapered, as described above, so as to progressively build up the compressive force that is applied to the absorbent collector head 12 as it is inserted down the inside of the tube, thereby squeezing out the liquid content from the absorbent head 12. Region 22a, at the bottom of the body (i.e. at the distal end of the body 22 from the closure portion 24), being narrowest, is configured to provide the greatest level of compression to the collector head 12. As shown in Figure 10, the wall thickness of the collection tube 20 may be increased in this region 22a, to enhance its ability to compress the collector head 12.

Conversely, the wall thickness of the collection tube 20 may be less in region 22b, to facilitate the application of gentle squeezing pressure in this region by the user when the collected fluid is being dispensed. To encourage the user to squeeze in this region rather than elsewhere, circular or elliptical regions 27 may be formed on the surface of the collection tube 20. The circular or elliptical regions 27 may be differentiated from the rest of the surface of the collection tube by being patterned or textured in some manner (e.g. by being frosted or hatched). Regions 27 are not the only places where the user may squeeze the collection tube 20 to dispense the fluid, and merely serve as a guide to the user where to do this.

Thus, in effect, the tapered collection tube 20 exhibits two different properties. The lower section 22a is rigidly firm so as to exert a high compressive force on the absorbent head 12 at the point of full insertion. The middle section 22b however is flexible, to facilitate squeezing by the user and thus the expulsion of fluid, and thereby causing the collection tube 20 to serve as a multifunctioning part.

Figure 11 shows a side view of the fully closed assembly 9, with closure part 16 of swab part 10 having been fully screwed onto closure portion 24 of collection tube 20, such that ribs 16a on closure part 16 align with ribs 25a on the shoulder 25 of collection tube 20.

*The release of fluid from the absorbent head upon the absorbent head reaching a maximum degree of compression at the bottom of the collection tube, and the associated flushing effect through the absorbent head*
As those skilled in the art will appreciate from the foregoing, the fluid collection device according to the above embodiments aims to release (or "elute"), from the absorbent collector head 12, a maximum available quantity of fluid collected from a sample environment. This is particularly important for situations in which the sample fluid may be less abundant, such as when collecting from the mouth of a dehydrated or young patient who may not produce much oral fluid/saliva.

Achieving release of a maximum available quantity of fluid from the absorbent head 12 is aided by a number of features cooperating synergistically: The absorbent head 12 is progressively compressed on insertion into the collection tube 20, as a result of the internal cross-sectional area of the collection tube 20 progressively decreasing (i.e. tapering) along the length of the collection tube 20, towards the end of the collection tube 20 furthest from its opening. With regard to the swab part 10, the length of the shaft 14, and thus the position of the absorbent head 12 relative to the closure part 16, is such that, when the closure part 16 is engaged with the closure portion 24 of the collection tube 20, the absorbent head 12 is brought into a predetermined position at the end of the collection tube 20, furthest from its opening, at which the internal cross-sectional area of the collection tube 20 is at its smallest, such as to put the absorbent head 12 into a state of maximum compression. Furthermore, the act of engaging the closure part 16 fully with the closure portion 24 (in the presently-preferred embodiments by screwing together the closure part 16 and the closure portion 24) causes further fluid to be squeezed out of the absorbent head 12. Such a screwing mechanism gives the user a mechanical advantage, potentially allowing them to compress the absorbent head 12 by more than would otherwise be possible if a screwing mechanism were not provided, and thereby enabling more fluid to be released from the absorbent head 12.

Thus, at the point of full engagement of the closure part 16 with the closure portion 24, the absorbent head 12 is positioned at a specific location at the end of the collection tube 20, where a maximum and repeatable squeezing force is applied. Furthermore, providing a screw thread as the means of engagement of the closure part 16 with the closure portion 24 gives the user a mechanical advantage which allows the absorbent head 12 to be subjected to tighter constriction at the end of the collection tube 20, so as to squeeze the absorbent head 12 more strongly.

Additionally, the tapered design of the collection tube 20 provides a predetermined and consistent level of squeeze around the whole of the absorbent head 12, making the output of the fluid collection device repeatable, consistent, and easy to control.

The fluid held in the absorbent head 12 is expelled as it is sealed in the collection tube 20. When inverted, the fluid will run directly towards the dropper spout 17 from which it can be dispensed. Once the cap 18 is removed from the dropper spout 17, compression of the tube 20 will cause the fluid to be forced out of the container in a predictable manner.

Furthermore, it will be appreciated that, during normal use of the fluid collection device, as described above, the release of the fluid from the absorbent head 12, on insertion of the absorbent head 12 into the collection tube 20, is not a reversible process. That is to say, once fluid has been extracted from the absorbent head 12, as a result of insertion of the absorbent head 12 into the collection tube 20, the fluid will not be reabsorbed by the absorbent head 12, since the absorbent head 12 is not allowed to expand again. This is as a result of not needing to undo the closure part 16 from the closure portion 24 in order to dispense fluid, since, with the present device, fluid is dispensed via the separate dropper spout 17 instead.

Thus, the present fluid collection device expels fluid from the absorbent head 12 during closure and keeps the fluid outside of the absorbent material. This is advantageous, not only in facilitating subsequent rapid dispensing of the liquid via the dropper spout 17 (i.e. without any need to squeeze the absorbent head again), but also in that the user can readily and rapidly appreciate how much fluid has been collected by the device. Accordingly, in practice, if the user finds that insufficient fluid has been collected, he or she can try again to collect some more, e.g. before parting from a patient or other subject. Furthermore, the user need not waste time squeezing the device only to find that insufficient liquid has been collected.

With reference now to Figure 12, this further illustrates the synergy of actions obtained on insertion of the absorbent head 12 into the collection tube 20, and also an associated flushing effect obtained through the absorbent head 12.

As the user forces the swab 10 down into the tapered and closed-ended collection tube 20, the absorbent head 12 and shaft 14 are required to displace the air (or liquid, if a buffer fluid has been added) from the bottom of the container 20.

Due to the closed end and tapered shape of the collection tube 20, paired with the absorbent head 12 filling the cross sectional area of the collection tube 20 along much of the tube's length, in practice the displaced air or liquid is forced to travel up through the absorbent medium (e.g. sponge) of the absorbent head 12.

This pneumatic or hydraulic movement of displaced air or liquid, in the opposite direction to the travel of the swab, flushes out a higher proportion of the sample fluid (e.g. oral fluid) contained in the absorbent head 12 than if one were simply to compress the volume of the absorbent head 12 by squeezing it.

This flushing process is illustrated sequentially in stages (a), (b) and (c) of Figure 12, with, in each case, the downward arrow towards the bottom of the collection tube 20 representing the direction of travel of the swab, and the upwardly-pointing arrow representing the displaced air or liquid performing a flushing effect through the absorbent head 12. It should be noted that, with the present fluid collection device, the displaced air or liquid from the bottom of the collection tube 20 is confined to flush through the absorbent head 12 (due to the size of the absorbent head 12 relative to the internal cross-sectional area of the collection tube 20), rather than being able to flow around the outside of the absorbent head 12, thereby assisting in the release of the sample fluid from the absorbent head 12.

Further, with the present fluid collection device, the pneumatic or hydraulic flushing effect described about works in tandem with the compression forces exerted on the absorbent head 12 during its insertion into the collection tube 20 - and particularly at the point of full insertion, with the closure part 16 of the closure assembly 40 of the swab part 10 becoming fully engaged with (e.g. screwed together with) the closure portion 24 of the collection tube 20 - so as to expel a very high proportion of the collected fluid from the absorbent head 12. These two effects advantageously work in synergy with the fixed length of the shaft 14, held firmly by the closure assembly 40; and the closure part 16 fastening (e.g. screwing) down to a predefined point on the closure portion 24 of the collection tube 20, sealing in the eluted fluid at the point of maximum compression of the absorbent head 12. Moreover, in accordance with the presently-preferred embodiments, the compression forces to which the absorbent head 12 is subjected, at the point of full insertion into the collection tube 20, are advantageously enhanced as a result of the mechanical advantage obtained through the provision of screw threads on the closure part 16 and the closure portion 24.

In effect, the mechanical advantage provided by the screw threads means that greater compression of the absorbent head 12 can be achieved with less force on the part of the user, making the task easier to perform by the user.

Additionally, the length of the screw threads and their pitch are preferably arranged to require only a 180° rotation in order to reach full engagement, again making the task easier to perform in a single action by hand.

Both of the above aspects are of great benefit when the user is performing multiple tests, reducing the risk of a repetitive strain injury.

### Modifications, alternative embodiments and other variants

Detailed embodiments have been described above. As those skilled in the art will appreciate, a number of additional modifications and alternatives can be made to the above embodiments whilst still benefiting from the inventions embodied therein.

For example, in the embodiments described above, the closure part 16 of the swab part 10 engages with the closure portion 24 of the collection tube 20 by screwing together, the threads being such that closure portion 24 locates within closure part 16. In alternative embodiments, however, the threads may be arranged differently, such that closure portion 24 locates around closure part 16 (or part thereof).

Moreover, in other alternative embodiments, fastening means other than screw threads may be used. For example, the closure part of the swab part and the closure portion of the collection tube may be configured to push together. Alternatively, a clip mechanism may be employed to attach the closure part of the swab part to the closure portion of the collection tube.

In the embodiments described above, the internal cross-sectional area of the container (i.e. collection tube 20) becomes smaller along its length by virtue of the collection tube itself having a tapered shape. In alternative embodiments, however, the collection tube itself need not have a tapered shape. Instead, the property of the internal cross-sectional area of the collection tube becoming smaller along its length may be by virtue of the shape of the inner surface of the container wall(s), or by virtue of a suitably-shaped (e.g. tapered) moulding or insert being provided within the container. In such cases, the internal cross-sectional area of the container is then defined as the cross-sectional area bounded by such inner wall surface(s), moulding(s) or insert(s).

In the embodiments described above, the swab shaft 14 is attached to the closure assembly 40 by means of a plurality of radially-disposed gripping fins 17d. However, other means for attaching the closure to the shaft are also possible, such as by using adhesive or a threaded fastening arrangement, as those skilled in the art will appreciate.

Another possible arrangement for the attachment of the swab shaft to the closure is illustrated in Figures 13a-c. In the swab part 10' as shown, the closure 16' is provided with a shaft-holding formation 21 to the side of the dropper spout 17'. The shaft 40' is held (e.g. with a friction fit) within the formation 21. (Other ways of attaching the shaft to an appropriate shaft-holding formation, e.g. by using a screw thread, are also possible.) As shown, one or both of the shaft-holding formation 21 and the dropper spout 17' may be radially offset. A cap (not illustrated) may be provided on the dropper spout 17', in a similar manner as described above.

The ability of the shaft 40' and the absorbent collector 12' to be rotated during the insertion/compression process within the collection tube (e.g. a tube 20 as before) is not significantly affected if the shaft-holding formation 21 is radially offset, since the shaft 40' may flex slightly during the insertion process (e.g. by virtue of the shaft 40' being made of a plastics material).

With the arrangement shown in Figures 13a-c, fluid from within the collection tube can easily reach the dropper spout 17' to be dispensed. Such an arrangement may be particularly beneficial if the fluid is relatively thick or viscous.

With the arrangement illustrated in Figures 13a-c, the shaft-holding formation 21 protrudes outwardly from the closure 16', alongside the dropper spout 17'. However, in a further variant, the shaft-holding formation may be formed within the closure 16' (e.g. protruding inwardly, in the opposite direction from the dropper spout 17'), so that the shaft-holding formation is not externally visible when the closure is attached to the collection tube in use.

The present disclosure provides a fluid collection device comprising an elongate container having an opening at one end, and a swab that is insertable into the container through said opening. The swab comprises a shaft, an absorbent collector at or near a distal end of the shaft for acquiring a fluid sample in use, and a closure at or near a proximal end of the shaft. The container incorporates means for compressing the absorbent collector as the swab is inserted into the container in use, thereby causing the absorbent collector to release at least some of the fluid sample held therein. The closure is adapted to engage with the container and to close the opening of the container, with the absorbent collector being inside the container in a compressed state. The closure further comprises an outlet through which, in use, fluid within the container can be dispensed, subsequent to the closure having been engaged with the container.

The means for compressing the absorbent collector comprises one or more surfaces within the container against which the absorbent collector compresses.

Other means for compressing the absorbent collector within the container are also possible, such as one or more moving parts within the container which act upon the absorbent collector so as to compress it.

## Claims

1. A fluid collection device comprising an elongate container (20) having an opening at one end, and a swab (10) that is insertable into the container (20) through said opening, wherein:
the swab (10) comprises a shaft (14), an absorbent collector (12) at or near a distal end of the shaft (14) for acquiring a fluid sample in use, and a closure (16, 40) at or near a proximal end of the shaft (14), the closure (16, 40) being adapted to engage with the container (20) and to close the opening of the container with the absorbent collector (12) and the shaft (14) inside the container (20);
the internal cross-sectional area of the container (20) becomes smaller in a direction away from the opening; and
the closure (16, 40) further comprises an outlet (17a) through which, in use, fluid within the container (20) can be dispensed, subsequent to the closure (16, 40) having been engaged with the container (20);
**characterised in that** the container (20) tapers along its length and the internal cross-sectional area of the container (20) decreases smoothly along its length so as to cause the absorbent collector (12) to be progressively radially compressed as the swab (10) is inserted into the container (20) and the absorbent collector (12) is advanced along the length of the container (20) in use, thereby causing the absorbent collector (12) to release at least some of the fluid sample held therein.

2. A fluid collection device as claimed in claim 1, wherein the internal cross-sectional area of the container (20) decreases along its length in such a manner that, in use, the absorbent collector (12) is further radially compressed as the closure (16, 40) is engaged with the container (20).

3. A fluid collection device as claimed in claim 1 or claim 2, wherein the length of the swab (10) and the length of the container (20) are such that the absorbent collector (12) reaches a maximum degree of radial compression at the far end of the container (20) from the opening.

4. A fluid collection device as claimed in any preceding claim, wherein the closure (16, 40) comprises friction-fit means by which the shaft (14) is attached.

5. A fluid collection device as claimed in claim 4, wherein the friction-fit means comprises a plurality of radially-disposed gripping fins (17d).

6. A fluid collection device as claimed in any preceding claim, wherein the opening of the container (20) is provided with a screw thread (24), and the closure (16, 40) is provided with a complementary screw thread (16b).

7. A fluid collection device as claimed in any preceding claim, wherein the closure (16, 40) further comprises a spout (17) in which the outlet (17a) is formed;
optionally wherein the spout (17) enables the device to function as a dropper.

8. A fluid collection device as claimed in any preceding claim, wherein the closure (16, 40) further comprises means for reversibly closing and opening the outlet (17a).

9. A fluid collection device as claimed in claim 8, wherein the means for reversibly closing and opening the outlet (17a) comprises a removable cap (18);
optionally wherein the cap (18) is attached to the rest of the closure (16, 40) by a flexible tether (19);
optionally wherein the cap (18) comprises a protruding lip (18a);
and optionally wherein the closure (16, 40), including the cap (18) and the flexible tether (19), is formed as a unitary construction, for example by injection moulding of a plastics material.

10. A fluid collection device as claimed in claim 9, wherein the cap (18) comprises plug means (18b) configured to engage within the outlet (17a).

11. A fluid collection device as claimed in claim 9 or claim 10, wherein the cap (18) and/or closure (16, 40) comprise detent means for securing the cap (18) in place when it closes the outlet (17a).

12. A fluid collection device as claimed in any preceding claim, wherein the container (20) is formed of a squeezable plastics material, for example by injection moulding.

13. A fluid collection device as claimed in claim 12, wherein the thickness of the wall of the container (20) is greater around the far end of the container from the opening.

14. A fluid collection device as claimed in any preceding claim, for the collection of bodily fluids such as oral fluids, blood or urine.

15. A method of collecting and subsequently dispensing a fluid sample using a fluid collection device as claimed in any preceding claim, the method comprising:
using the absorbent collector (12) of the swab (10) to acquire the fluid sample;
extracting fluid from the absorbent collector (12) by inserting the swab (10) into the container (20) through the opening, and progressively radially compressing the absorbent collector (12) by advancing the absorbent collector (12) along the length of the container (20);
engaging the closure (16, 40) with the container (20), and thereby closing the opening of the container (20) with the absorbent collector (12) and the shaft (14) inside the container (20); and
dispensing a quantity of the fluid from the container (20) through the outlet (17a).

## Patentansprüche

1. Fluidsammelvorrichtung, die einen länglichen Behälter (20), der eine Öffnung an einem Ende aufweist, und einen Tupfer (10), der durch die Öffnung in den Behälter (20) eingeführt werden kann, umfasst, wobei:
der Tupfer (10) einen Schaft (14), einen Absorptionskollektor (12) an oder nahe einem distalen Ende des Schafts (14) zum Aufnehmen einer verwendeten Fluidprobe und einen Verschluss (16, 40) an oder in der Nähe eines proximalen Endes des Schafts (14) umfasst, wobei der Verschluss (16, 40) dafür ausgelegt ist, mit dem Behälter (20) in Eingriff zu treten und die Öffnung des Behälters mit dem Absorptionskollektor (12) und dem Schaft (14) innerhalb des Behälters (20) zu schließen;
die innere Querschnittsfläche des Behälters (20) in einer Richtung weg von der Öffnung kleiner wird; und
der Verschluss (16, 40) ferner einen Auslass (17a) umfasst, durch den bei Gebrauch Fluid innerhalb des Behälters (20) abgegeben werden kann, nachdem der Verschluss (16, 40) mit dem Behälter (20) in Eingriff gebracht worden ist;
**dadurch gekennzeichnet, dass** sich der Behälter (20) entlang seiner Länge verjüngt und die innere Querschnittsfläche des Behälters (20) entlang seiner Länge gleichmäßig abnimmt, um zu bewirken, dass der Absorptionskollektor (12) beim Einführen des Tupfers (10) in den Behälter (20) progressiv radial zusammengedrückt wird und der Absorptionskollektor (12) entlang der Länge des verwendeten Behälters (20) vorgeschoben wird, wodurch der Absorptionskollektor (12) mindestens einen Teil der darin enthaltenen Fluidprobe freisetzt.

2. Fluidsammelvorrichtung nach Anspruch 1, wobei die innere Querschnittsfläche des Behälters (20) entlang seiner Länge derart abnimmt, dass bei Verwendung der Absorptionskollektor (12) weiter radial zusammengedrückt wird, wenn der Verschluss (16, 40) mit dem Behälter (20) in Eingriff gebracht wird.

3. Fluidsammelvorrichtung nach Anspruch 1 oder Anspruch 2; wobei die Länge des Tupfers (10) und die Länge des Behälters (20) so beschaffen sind, dass der Absorptionskollektor (12) am anderen Ende des Behälters (20) von der Öffnung ein maximales Ausmaß an radialer Kompression erreicht.

4. Fluidsammelvorrichtung nach einem der vorhergehenden Ansprüche, wobei der Verschluss (16, 40) Reibungspassmittel umfasst, mit denen der Schaft (14) befestigt ist.

5. Fluidsammelvorrichtung nach Anspruch 4, wobei das Reibungspassmittel mehrere radial angeordnete Greifrippen (17d) umfasst.

6. Fluidsammelvorrichtung nach einem der vorhergehenden Ansprüche, wobei die Öffnung des Behälters (20) mit einem Schraubengewinde (24) versehen ist und der Verschluss (16, 40) mit einem komplementären Schraubengewinde (16b) versehen ist.

7. Fluidsammelvorrichtung nach einem der vorhergehenden Ansprüche, wobei der Verschluss (16, 40) ferner einen Auslauf (17) umfasst, in dem der Auslass (17a) gebildet ist;
wobei der Auslauf (17) optional der Vorrichtung ermöglicht, als Tropfer zu fungieren.

8. Fluidsammelvorrichtung nach einem der vorhergehenden Ansprüche, wobei der Verschluss (16, 40) ferner Mittel zum reversiblen Schließen und Öffnen des Auslasses (17a) umfasst.

9. Fluidsammelvorrichtung nach Anspruch 8, wobei das Mittel zum reversiblen Schließen und Öffnen des Auslasses (17a) eine entfernbare Kappe (18) umfasst;
optional wobei die Kappe (18) durch einen flexiblen Haltegurt (19) am Rest des Verschlusses (16, 40) befestigt ist;
optional wobei die Kappe (18) eine hervorstehende Lippe (18a) umfasst;
und optional wobei der Verschluss (16, 40) einschließlich der Kappe (18) und des flexiblen Haltegurts (19) als einheitliche Konstruktion, beispielsweise durch Spritzgießen eines Kunststoffmaterials, gebildet ist.

10. Fluidsammelvorrichtung nach Anspruch 9, wobei die Kappe (18) Stopfenmittel (18b) umfasst, die dafür konfiguriert sind, in den Auslass (17a) einzugreifen.

11. Fluidsammelvorrichtung nach Anspruch 9 oder Anspruch 10, wobei die Kappe (18) und/oder der Verschluss (16, 40) Rastmittel zum Befestigen der Kappe (18) an Ort und Stelle umfassen, wenn sie den Auslass (17a) schließt.

12. Fluidsammelvorrichtung nach einem der vorhergehenden Ansprüche, wobei der Behälter (20) aus einem zusammendrückbaren Kunststoffmaterial beispielsweise durch Spritzgießen gebildet ist.

13. Fluidsammelvorrichtung nach Anspruch 12, wobei die Dicke der Wand des Behälters (20) um das von der Öffnung entfernte Ende des Behälters größer ist.

14. Fluidsammelvorrichtung nach einem der vorhergehenden Ansprüche zum Sammeln von Körperflüssigkeiten wie Mundflüssigkeiten, Blut oder Urin.

15. Verfahren zum Sammeln und anschließenden Abgeben einer Fluidprobe unter Verwendung einer Fluidsammelvorrichtung nach einem der vorhergehenden Ansprüche, wobei das Verfahren Folgendes umfasst:
Verwenden des Absorptionskollektors (12) des Tupfers (10) zum Aufnehmen der Fluidprobe;
Extrahieren von Fluid aus dem Absorptionskollektor (12) durch Einführen des Tupfers (10) in den Behälter (20) durch die Öffnung, und fortschreitendes radiales Zusammendrücken des Absorptionskollektors (12) durch Vorschieben des Absorptionskollektors (12) entlang der Länge des Behälters (20);
Eingreifen des Verschlusses (16, 40) in den Behälter (20) und dadurch Schließen der Öffnung des Behälters (20) mit dem Absorptionskollektor (12) und dem Schaft (14) innerhalb des Behälters (20); und
Abgeben einer Fluidmenge aus dem Behälter (20) durch den Auslass (17a).

## Revendications

1. Dispositif de prélèvement de fluide comprenant un contenant allongé (20) ayant une ouverture au niveau d'une extrémité, et un écouvillon (10) qui peut être inséré jusque dans le contenant (20) à travers ladite ouverture, dans lequel :
l'écouvillon (10) comprend une tige (14), un collecteur absorbant (12) au niveau ou près d'une extrémité distale de la tige (14) destiné à acquérir un échantillon de fluide pendant l'utilisation, et une fermeture (16, 40) au niveau ou près d'une extrémité proximale de la tige (14), la fermeture (16, 40) étant conçue pour entrer en prise avec le contenant (20) et pour fermer l'ouverture du contenant avec le collecteur absorbant (12) et la tige (14) à l'intérieur du contenant (20) ;
la surface de section transversale interne du contenant (20) rapetisse dans une direction éloignée de l'ouverture ; et
la fermeture (16, 40) comprend en outre une sortie (17a) par laquelle, pendant l'utilisation, du fluide au sein du contenant (20) peut être délivré, suite à l'entrée en prise de la fermeture (16, 40) avec le contenant (20) ;
**caractérisé en ce que** le contenant (20) s'effile le long de sa longueur et la surface de section transversale interne du contenant (20) diminue doucement le long de sa longueur de manière à entraîner la compression radiale progressive du collecteur absorbant (12) quand l'écouvillon (10) est inséré jusque dans le contenant (20) et le collecteur absorbant (12) est avancé le long de la longueur du contenant (20) pendant l'utilisation, amenant ainsi le collecteur absorbant (12) à libérer au moins une partie de l'échantillon de fluide contenu dans celui-ci.

2. Dispositif de prélèvement de fluide selon la revendication 1, dans lequel la surface de section transversale du contenant (20) diminue le long de sa longueur de telle manière que, pendant l'utilisation, le collecteur absorbant (12) est comprimé davantage de manière radiale quand la fermeture (16, 40) entre en prise avec le contenant (20).

3. Dispositif de prélèvement de fluide selon la revendication 1 ou la revendication 2, dans lequel la longueur de l'écouvillon (10) et la longueur du contenant (20) sont telles que le collecteur absorbant (12) atteint un degré maximal de compression radiale au niveau de l'extrémité du contenant (20) éloignée de l'ouverture.

4. Dispositif de prélèvement de fluide selon l'une quelconque des revendications précédentes, dans lequel la fermeture (16, 40) comprend un moyen d'ajustement par frottement par lequel la tige (14) est attachée.

5. Dispositif de prélèvement de fluide selon la revendication 4, dans lequel le moyen d'ajustement par frottement comprend une pluralité d'ailettes de préhension disposées de manière radiale (17d).

6. Dispositif de prélèvement de fluide selon l'une quelconque des revendications précédentes, dans lequel l'ouverture du contenant (20) est pourvue d'un filetage de vis (24), et la fermeture (16, 40) est pourvue d'un filetage de vis complémentaire (16b).

7. Dispositif de prélèvement de fluide selon l'une quelconque des revendications précédentes, dans lequel la fermeture (16, 40) comprend en outre un bec (17) dans lequel est formée la sortie (17a) ;
facultativement dans lequel le bec (17) permet au dispositif de fonctionner comme un compte-gouttes.

8. Dispositif de prélèvement de fluide selon l'une quelconque des revendications précédentes, dans lequel la fermeture (16, 40) comprend en outre un moyen destiné à fermer et à ouvrir la sortie de manière réversible (17a).

9. Dispositif de prélèvement de fluide selon la revendication 8, dans lequel le moyen destiné à fermer et à ouvrir la sortie de manière réversible (17a) comprend un capuchon amovible (18) ;
facultativement dans lequel le capuchon (18) est attaché au reste de la fermeture (16, 40) par une attache souple (19) ;
facultativement dans lequel le capuchon (18) comprend une lèvre faisant saillie (18a) ;
et facultativement dans lequel la fermeture (16, 40), incluant le capuchon (18) et l'attache souple (19), est formée comme une construction unitaire, par exemple par moulage par injection d'une matière plastique.

10. Dispositif de prélèvement de fluide selon la revendication 9, dans lequel le capuchon (18) comprend un moyen d'obturation (18b) configuré pour entrer en prise au sein de la sortie (17a).

11. Dispositif de prélèvement de fluide selon la revendication 9 ou la revendication 10, dans lequel le capuchon (18) et/ou la fermeture (16, 40) comprennent un moyen de détente destiné à fixer le capuchon (18) en place quand il ferme la sortie (17a).

12. Dispositif de prélèvement de fluide selon l'une quelconque des revendications précédentes, dans lequel le contenant (20) est formé d'une matière plastique pouvant être pressée, par exemple par moulage par injection.

13. Dispositif de prélèvement de fluide selon la revendication 12, dans lequel l'épaisseur de la paroi du contenant (20) est supérieure autour de l'extrémité du contenant (20) éloignée de l'ouverture.

14. Dispositif de prélèvement de fluide selon l'une quelconque des revendications précédentes, pour le prélèvement de fluides corporels tels que les fluides oraux, le sang ou l'urine.

15. Procédé de prélèvement et puis de délivrance d'un échantillon de fluide en utilisant le dispositif de prélèvement de fluide selon l'une quelconque des revendications précédentes, le procédé comprenant :
l'utilisation du collecteur absorbant (12) de l'écouvillon (10) pour acquérir l'échantillon de fluide ;
l'extraction du fluide à partir du collecteur absorbant (12) en insérant l'écouvillon (10) jusque dans le récipient (20) à travers l'ouverture, et en comprimant progressivement de manière radiale le collecteur absorbant (12) en faisant avancer le collecteur absorbant (12) le long de la longueur du contenant (20) ;
l'entrée en prise de la fermeture (16, 40) avec le contenant (20), et ainsi la fermeture de l'ouverture du contenant (20) avec le collecteur absorbant (12) et la tige (14) à l'intérieur du contenant (20) ; et
la délivrance d'une quantité du fluide du contenant (20) à travers la sortie (17a).
